# EUROPEAN PATENT APPLICATION

(11) **EP 2 169 524 A2**
(43) Date of publication of application: **31.03.2010**
(21) Application number: 09012335.7
(22) Date of filing: 29.09.2009
(51) Int. Cl.: G06F 3/048

(54) **Apparatus, method and program for controlling drag and drop operation and computer terminal**

(30) Priority: 30.09.2008 JP 2008252447
(71) Applicant: FUJIFILM Corporation, Minato-ku Tokyo (JP)
(72) Inventor: Takeshita, Masanori, Tokyo (JP)
(74) Representative: Klunker . Schmitt-Nilson . Hirsch

(57) **Abstract**

Multiple displays (37a, 37b, 37c) are placed side by side. A key image (58a, 58b) to be drag-and-dropped is displayed on a first display (37a). An actual drop target area (56) into which the key image is to be dropped is displayed on the rest of the displays. The key image is held by placing a cursor thereon and pressing a right click button (42b) of a mouse (42). In response to this, a drop window (60) that is a reduced image of the drop target area is displayed on the first display. The cursor is moved onto the drop window by operating the mouse with the right click button pressed. Releasing the right click button in a desired position in the drop window drops the key image. Based on a drop position in the drop window, the key image is dropped in a corresponding position in the actual drop target area.

## Description

### FIELD OF THE INVENTION

The present invention relates to an apparatus, a method and a program for controlling a drag and drop operation to move or copy an object displayed on a screen and a computer terminal.

### BACKGROUND OF THE INVENTION

Recently, various operating systems such as Windows (registered trademark) have a so-called multi-display function, that is, a function to control multiple displays using one computer. The multi-display function enables the computer to display a single image across two or more displays, or a different image on each of the displays. With the use of the multi-display function, a cursor that selects an object on a screen moves across the multiple displays in accordance with the operation of a pointing device such as a mouse.

For example, in a medical information system, a multi-display terminal having at least two displays is used. On the displays are displayed separately an interpretation report of an examination image interpreted by a doctor specialized in diagnostic imaging (hereinafter referred to interpretation doctor) and an image viewer that displays the examination image. While viewing the interpretation report using the multi-display terminal, a user can select an examination image (key image) attached to the interpretation report using a cursor, and drag and drop the key image in the image viewer displayed on another display. Thereby, the key image is enlarged and displayed in the image viewer.

To move the cursor across the multiple displays, a mouse needs to be moved across a long distance. As a result, operability is impaired, which likely to cause fatigue to a user. To reduce a mouse drag distance, in Japanese Patent No. 3566916, a cursor is moved to the same position on a different display by a predetermined operation other than an operation to move the cursor. In Japanese Patent No. 3646860, a cursor is instantly moved to a different window in accordance with wheel operation of an intelligent mouse. In U.S. Patent No. 5,635,954 corresponding to Japanese Patent No. 3168156, a moving direction of a mouse is detected when a predetermined button of the mouse is pressed, and a cursor is moved to a point on a screen according to the detected moving direction.

In Japanese Patents No. 3566916 and 3646860 and U.S. Patent No. 5, 635, 954, the mouse drag distance is reduced by additional operations other than the operation to move the cursor. However, it is not preferable to add operations to the drag and drop operation that requires the operation to select and move the object.

Normally, in a drag and drop operation, a cursor is placed on an object, and a mouse is moved with a click button pressed, and then the click button is released at a desired position. If an operation other than the cursor-moving operation is added for the purpose of reducing the mouse drag distance, it makes the drag and drop operation complicated and impairs operability.

Cursor moving methods disclosed in Japanese Patents No. 3566916 and 3646860 and U.S. Patent No. 5, 635, 954 only intend to improve the operability of moving a cursor, but not the operability of the drag and drop operation.

### SUMMARY OF THE INVENTION

An object of the present invention is to provide an apparatus, a method and a program for improving operability of a drag and drop operation and a computer terminal.

In order to achieve the above and other objects, an apparatus for controlling a drag and drop operation of the present invention includes a display section, a drop window display controller, an operating section, a drag and drop processor, and a drop position controller. The display section displays an object. The drop window display controller displays a drop window on the display section. The drop window corresponds to an actual drop target area into which the object is to be dropped. The drop window has a smaller size than the actual drop target area, and is displayed in a position closer to the object than the actual drop target area. The operating section instructs holding, dragging and dropping of the object. The drag and drop processor drags the object and drops the object in the drop window based on the operation of the operating section. The drop position controller controls a corresponding position in the actual drop target area into which the object is to be dropped based on a drop position of the object in the drop window.

It is preferable that the object is held, dragged and dropped via a cursor displayed on the display section.

It is preferable that the object dropped in the actual drop target area is displayed in a large size.

It is preferable that the drop window display controller displays the drop window when the operating section provides a predetermined instruction. When the drop window is not displayed, the actual drop target area is the only area into which the object is to be dropped.

It is preferable that the drop window display controller displays the drop window when the object is held, and hides the drop window when the object is dropped in the drop window.

It is preferable that the display section has multiple displays placed side by side, and the actual drop target area is displayed across the displays, and the drop window is displayed on one of the displays where the object is displayed.

It is preferable that the actual drop target area has a plurality of divided areas.

It is preferable that the drop window is a reduced image of the actual drop target area.

It is preferable that the apparatus for controlling the drag and drop operation has a position information display section for displaying position information. The position information indicates a position in the actual drop target area, corresponding to a position in the drop window into which the object is dropped.

It is preferable that the position information display section displays a pseudo cursor in the actual drop target area on the position indicated by the position information.

It is preferable that the position information is coordinates.

A method for controlling a drag and drop operation includes a first display step, a second display step, an operating step, a dragging and dropping step, and a dropping step. In the first display step, an object is displayed on a display section. In the second display step, a drop window is displayed on the display section. The drop window corresponds to an actual drop target area into which the object is to be dropped. The drop window has a smaller size than the actual drop target area, and is displayed in a position closer to the object than the actual drop target area. In the operating step, an operating section is operated to instruct holding, dragging and dropping of the object. In the dragging and dropping step, the object is dragged and then dropped inside the drop window based on the operation of the operating section. In the dropping step, the object is dropped in a corresponding position in the actual drop target area based on a drop position of the object in the drop window.

A drag and drop control program of the present invention causes the computer to execute the method for controlling the drag and drop operation of the present invention.

A computer terminal of the present invention includes a computer main unit for executing the program for controlling the drag and drop operation, the operating section and the display section on which the object is displayed.

It is preferable that the drop window is displayed on the display section when a predetermined instruction is provided by the operating section.

According to the present invention, the drop window that is the reduced image of the actual drop target area is displayed in a position closer to the object than the actual drop target area. When the object is dropped in the drop window, the object is dropped in a position inside the actual drop target area, corresponding to the drop position of the object in the drop window. Accordingly, the drag distance is reduced without complicating the operation, and thus the operability of the drag and drop operation is improved.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Figure 1 is a configuration diagram of a medical information system;
Figure 2 is an internal block diagram of a computer used as a clinical terminal, a report creation terminal, or a DB server;
Figure 3 is a schematic view of the clinical terminal;
Figure 4 is an explanatory view of a CPU of the clinical terminal and an explanatory view of a display screen on each display;
Figure 5 is an explanatory view of a drop window;
Figure 6 is an explanatory view of a display screen after a drop operation;
Figure 7 is a flow chart illustrating steps to control a drag and drop operation;
Figure 8 is a modified example of the drop window;
Figure 9 is another modified example of the drop window; and
Figure 10 is an explanatory view of an example in which a drop position is clearly indicated by coordinates.

### DESCRIPTION OF THE PREFERRED EMBODIMENTS

A medical information system, provided in a medical facility such as a hospital, supports creating interpretation reports (or simply referred to as reports) and viewing them by managing the created reports in a retrievable manner. The reports describe interpretation of examination images taken with medical modalities such as a CR (Computed Radiography) device, a CT (Computed Tomography) device and a MRI (Magnetic Resonance Imaging) device. The reports are created by, for example, radiologists who are specialized in diagnostic imaging. Hereinafter, a medical information system according to the present invention is described.

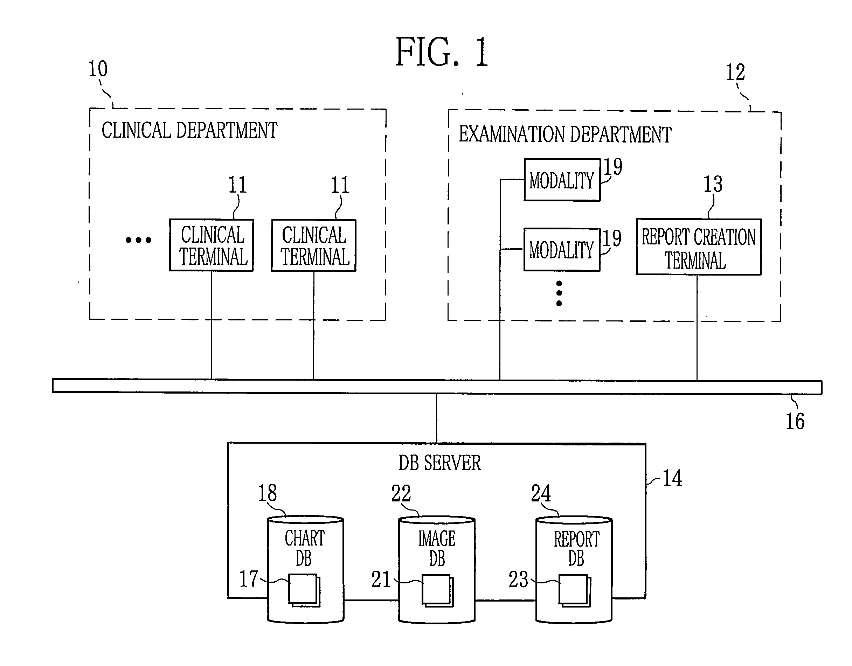

In Fig. 1, a medical information system is composed of a clinical terminal 11 installed in a clinical department 10, a report creation terminal 13 installed in a radiological examination department (hereinafter simply referred to as examination department) 12 and a database (DB) server 14. These are communicably connected via a network 16. The network 16 is, for example, a LAN (Local Area Network) provided in a hospital.

In the DB server 14 are constructed a plurality of databases such as a chart database (DB) 18 for storing data of medical charts (hereinafter referred to as charts) 17 or medical records (progress notes) of the patients, an image database (DB) 22 for storing data of examination images 21 taken with modalities 19, a report database (DB) 24 for storing data of interpretation reports (hereinafter referred to as reports) 23 created using the report creation terminals 13. The examination images 21 include radioscopic images taken with the CR devices, tomographic images taken with the CT and the MRI devices, and three-dimensional images generated based on the tomographic images.

The DB server 14 functions as a so-called PACS (Picture Archiving and Communication Systems) server that receives the data of the examination images 21 from the modality 19 via the network 16, and stores it in the image DB 22. The PACS is composed of the DB server 14 and the modality 19. The data of the examination images 21 is stored in a file format compliant with DICOM (Digital Imaging and Communications in Medicine), for example. A DICOM tag is attached to the file. The DICOM tag contains patient record information such as a patient ID, an examination ID, an examination date, a type of examination and the like.

A chart system is composed of the DB server 14, the clinical terminal 11 and the chart DB 18. A report system is composed of the DB server 14, the report creation terminal 13, the image DB 22 and the report DB 24. Data of the reports 23 is stored in the report DB 24 in a searchable manner. An examination ID, a patient ID, a name of a patient or the like can be used as search keys.

The clinical terminal 11 is operated by a doctor in the clinical department 10, and used for viewing or inputting charts 17, and issuing a request for an examination to the examination department 12. The clinical terminal 11 also functions as a report viewing terminal that displays the requested examination images 21 and the reports 23 provided by the examination department 12. For this reason, the clinical terminal 11 constitutes the above-described report system together with the report creation terminal 13, the image DB 22 and the report DB 24.

When the request is issued by the clinical terminal 11, the request is transmitted to and accepted by a request reception terminal (not shown) installed in the examination department 12. The request reception terminal attaches an examination ID to the received request to manage the request data. The examination ID is transmitted back to the clinical terminal 11 together with the notice of receipt of the request. In a case interpretation is required, the request with the examination ID is then transmitted from the request reception terminal to the report creation terminal 13. Based on the request, a staff in the examination department 12 takes an examination image using the modality 19.

The radiologist checks the request on the report creation terminal 13. In a case interpretation is required, the radiologist retrieves data of the examination images 21 to be interpreted from the image DB 22. Findings from the interpretation are written in the report 23, and the examination images 21 related to the findings are attached to the report 23 as key images. Data of the created report 23 is stored in the report DB 24. When the creation of the report 23 is completed, a notice of completion is transmitted from the report creation terminal 13 to the clinical terminal 11 that issued the request. The notice of completion contains addresses to access the requested data of the examination images 21 stored in the image DB 22 and the reports 23 stored in the report DB 24. The doctor accesses the addresses via the clinical terminal 11 and views the requested examination images 21 and the reports 23.

Each of terminals 11 and 13 and the DB server 14 is a computer such as a personal computer, a server computer or a work station. The computer is installed with a control program such as an operating system, and an application program such as a client program and a server program.

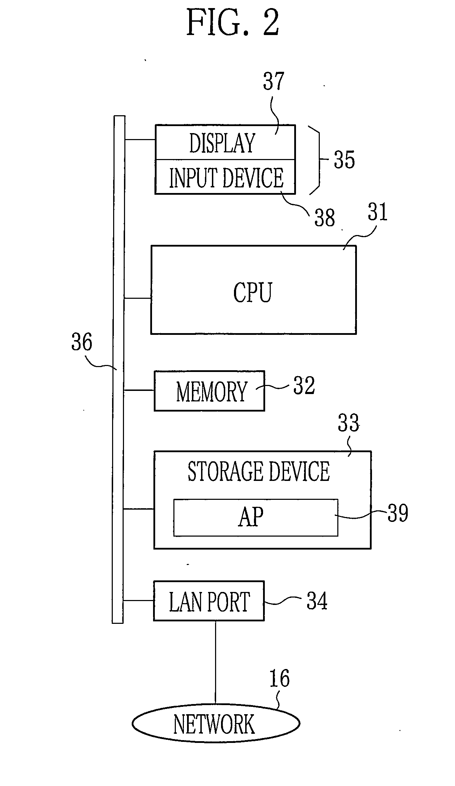

As shown in Fig. 2, the computers used as the terminals 11 and 13 and the DB server 14 have the same basic configuration. Each computer is provided with a CPU 31, a memory 32, a storage device 33, a LAN port 34 and a console 35, and these components are interconnected via a data bus 36. The console 35 is composed of a display 37 and an input device (operating section) 38 such as a keyboard and a mouse.

The storage device 33 is an HDD (Hard Disk Drive), for example, and stores a control program, an application program (hereinafter abbreviated as AP) 39 and the like. A server in which a database is built is provided with the storage device 33 for the database, for example, a disk array in which a plurality of the HDDs are connected, separately from the HDD that stores the programs.

The memory 32 is a work memory where the CPU 31 executes processing. The CPU 31 loads the control program stored in the storage device 33 to the memory 32, and executes processing based on the control program. Thus, the CPU 31 controls the entire computer. The LAN port 34 is a network interface that controls transmission via the network 16.

The clinical terminal 11 is installed with a client program as the AP 39, such as chart software for viewing and editing the charts 17 and viewer software for viewing the examination images 21 and the reports 23. When the client program starts, an operation screen with GUI (Graphical User Interface) is displayed on the display of the clinical terminal 11. The operation screen includes display screens displaying the chart 17 retrieved from the chart DB 18, the examination image 21 retrieved from the image DB 22 and the report 23 retrieved from the report DB 24. The client program includes a drag and drop control program for performing drag and drop operations of the objects, such as the examination image 21 displayed on the display, with the operation of a mouse as will be described later.

Operating instructions to input and edit the chart 17 or to input and issue a request are entered into the clinical terminal via the console 35. The input charts 17 and the request data are stored in the chart DB 18.

The DB server 14 is installed with a server program as the AP 39. The server program executes processing in response to a request of a client and sends back the result of the processing to the client. The CPU of the DB server 14 functions as a storage processor and a search processor for data (the charts 17, the examination images 21 and the reports 23) by executing the server program. In response to a request for data storage from a client such as the clinical terminal 11, the modality 19 or the report creation terminal 13, the storage processor stores the charts 17 in the chart DB 18, the examination images 21 in the image DB 22, and the reports 23 in the report DB 24. In response to a delivery request sent from the clinical terminal 11 or the report creation terminal 13 for the charts 17, the examination images 21 or the reports 23, the search processor retrieves the requested data from the chart DB 18, the image DB 22 or the report DB 24, and delivers the retrieved data to the clinical terminal 11 or the report creation terminal 13 that issued the delivery request.

The report creation terminal 13 is installed with a client program as the AP 39. The client program is a report creation support program for editing reports. The report creation terminal 13 performs display processing of the examination images 21 in addition to the edit processing of the report 23 by executing the client program. The report creation terminal 13 has a function to display the created report 23. Similar to the clinical terminal 11, the report creation terminal 13 also functions as the report viewing terminal. It should be noted that this client program also includes a drag and drop control program to drag and drop an object, such as the examination image 21 displayed on the display, with the operation of the mouse as will be described later.

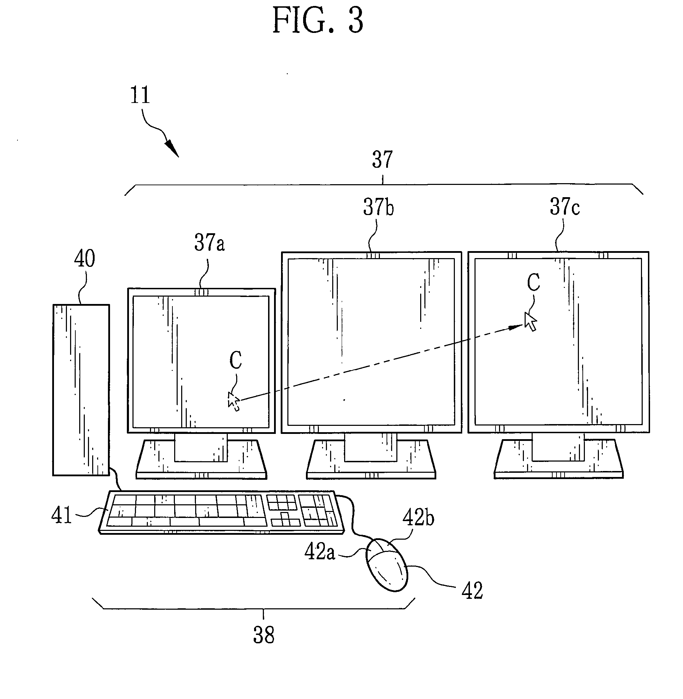

As shown in Fig. 3, the clinical terminal 11 is provided with a main unit 40 of the computer incorporating the CPU 31, the memory 32, the storage device 33 and the LAN port 34. The display 37 has a multi-display configuration using three displays 37a, 37b, and 37c all connected to the main unit 40. In addition, a keyboard 41 and a mouse 42 as the input devices 38 are connected to the main unit 40.

The chart 17, the report 23 and a request input screen are displayed on the display 37a. The displays 37b and 37c have higher resolution than the display 37a. The examination images 21 are displayed on the displays 37b and 37c. The display 37a has the resolution of, for example, 1280 x 1024. The displays 37b and 37c have the resolution of, for example, 2560 x 2048. It should be noted that the report creation terminal 13 has a configuration similar to the clinical terminal 11.

A position indicator, for example, a cursor C displayed on one of the displays 37a to 37c is moved in accordance with the operation of the mouse 42. As is well known, the mouse 42 has an X-axis roller and a Y-axis roller, and a moving distance of the mouse 42 in each axis direction is obtained from a rotation amount of each roller. The moving distance of the mouse 42 is sent to the CPU 31 as coordinate position data of the cursor C.

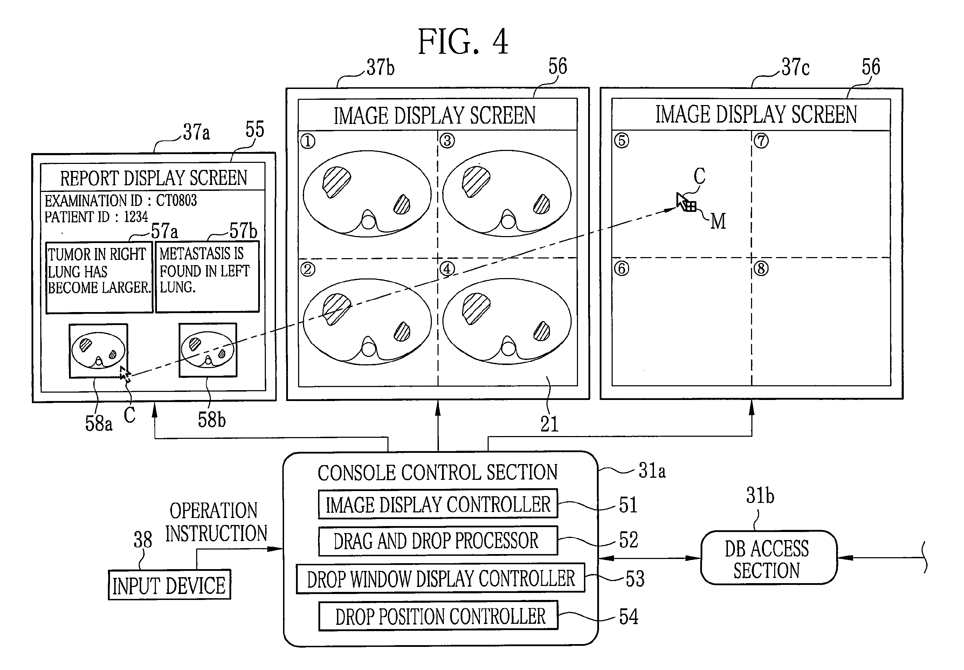

As shown in Fig. 4, in a case that the clinical terminal 11 or the report creation terminal 13 functions as the report viewing terminal, a viewer software used for viewing the examination images 21 and the reports 23 is started. Thereby, the CPU 31 (see Fig. 2) functions as a console control section 31a or user interface control section for drag and drop operation and a DB access section 31b. The console control section 31a is provided with an image display controller 51, a drag and drop processor 52, a drop window display controller 53 and a drop position controller 54.

The DB access section 31b receives data of the report 23 from the report DB 24. The image display controller 51 generates a report display screen 55 used for viewing the received report 23, and outputs the generated report display screen 55 to the display 37a. The image display controller 51 generates image display screens 56 used for observing the examination images 21, and outputs the generated image display screens 56 to the displays 37a and 37c respectively.

The report display screen 55 displays basic information such as the examination ID and the patient ID contained in the request. In addition, the report display screen 55 is provided with boxes 57a and 57b for displaying the findings input by the radiologist. Below the boxes 57a and 57b, the examination images 21 related to the findings described in the boxes 57a and 57b are displayed as key images 58a and 58b. The key images 58a and 58b are reduced images of the examination images 21.

Each of the two image display screens 56 is divided into four divided areas to arrange and display four images. The displays 37b and 37c altogether are divided into eight divided areas. Numbers one to eight are assigned to the divided areas respectively for identification. The key images 58a and 58b in the report display screen 55 can be drag-and-dropped with the operation of the mouse 42. In the divided areas No. 1 to No. 8, the examination images 21 corresponding to the key images drag-and-dropped from the report display screen 55 are displayed in a large size.

The report display screen 55 and the image display screens 56 are operation screens using GUI. The console control section 31a outputs the operation screens to the displays 37a to 37c, and accepts operating instructions from the input device 38 via the operation screens.

The coordinate position data is successively input to the console control section 31a with the use of the mouse 42. Based on this coordinate position data, the console control section 31a successively determines the position of the cursor C to be displayed on one of the displays 37a to 37c. The image display controller 51 displays the cursor C in a position determined by the console control section 31a. In accordance with the movement of the mouse 42, the cursor C moves across the displays 37a to 37c, and is displayed on one of the report display screen 55 and the image display screens 56.

The mouse 42 is provided with a left click button 42a and a right click button 42b (see Fig. 3) for selecting an object such as the key image 58a or 58b displayed on one of the displays 37a to 37c using the cursor C. A click signal is input from the mouse 42 to the console control section 31a. The console control section 31a judges whether the left click button 42a or the right click button 42b is clicked based on the input click signal.

The drag and drop processor 52 performs normal direct drag and drop processing and indirect drag and drop processing. In the direct drag and drop processing, the key image is held (selected) when the cursor C is placed on the key image and the left click button 42a of the mouse 42 is pressed (clicked). By moving the mouse 42 across a long distance with the left click button 42a pressed, the key image held by the cursor C is dragged to one of the divided areas (drop target areas) No. 1 to No. 8 of the two image display screens 56. Thereafter, releasing the left click button 42a drops the key image in the drop target area. In the divided area where the key image has been dropped, the examination image 21 corresponding to the key image is displayed in a large size. It should be noted that during the drag operation, the key image being dragged is indicated by a mark M, and this mark M is attached to the cursor C.

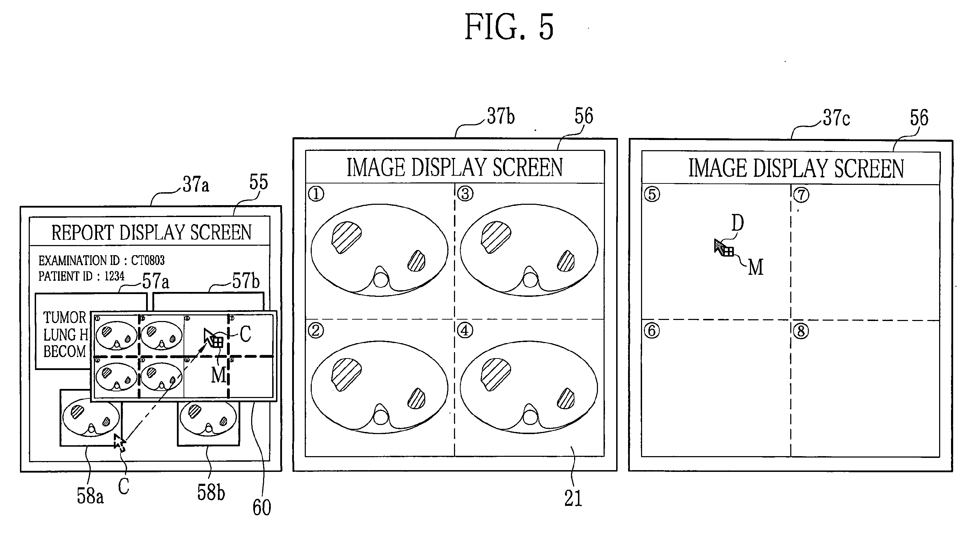

In the indirect drag and drop processing, the key image is held (selected) when the cursor C is placed on the key image and the right click button 42b of the mouse 42 is pressed. In response to this, as shown in Fig. 5, the drop window display controller 53 displays a drop window 60 or control window area in a pop-up display in a position close to the cursor C inside the report display screen 55. The mouse 42 is operated with the right click button 42b pressed to drag the key image held by the cursor C into the drop window 60. Releasing the right click button 42b in a desired position on the drop window 60 drops the key image.

The drop position controller 54 detects the drop position of the key image on the drop window 60, and displays the examination image corresponding to the key image in a large size in the divided area on the image display screen 56, corresponding to the drop position. In response to this, the drop window display controller 53 hides the drop window 60 on the report display screen 55.

The drop window 60 displays in a list form the available divided areas into which the key image is to be dropped. Since the drop window 60 is smaller than the drop target area in size, the drag distance is significantly reduced, and thus the operation is facilitated. In this example, the drop window 60 is a reduced combined image of the divided areas No. 1 to No. 8 of the two image display screens 56.

As shown in Fig. 5, when the key image 58a is dragged into the drop window 60, the image display controller 51 displays a pseudo cursor D on the image display screen 56 that is the drop target area or active area. The pseudo cursor D is located at coordinates corresponding to those of the cursor C on the drop window 60. The pseudo cursor D indicates an actual position or target location into which the key image is to be dropped. The image display controller 51 hides the pseudo cursor D when hiding the drop window 60. Thus, the image display controller 51 functions as position information display section that displays position information of the actual position into which the key image is to be dropped.

It is preferable that the cursor C and the pseudo cursor D are displayed differently from each other for the sake of visual distinction. It is preferable to display them in different colors, for example, the cursor C is displayed white, and the pseudo cursor D is displayed gray. It is also preferable to change transparency of the pseudo cursor D to translucent, or to change the shape of the pseudo cursor D from that of the cursor C. Thus, an operator clearly identifies a position in the image display screen 56 into which the examination image 21 is actually dropped.

Next, an operation of the above configuration is described. A doctor issues a request using the clinical terminal 11. The report creation terminal 13 receives the request issued from the clinical terminal 11 via the request reception terminal in the examination department 12. A radiologist checks the request on the report creation terminal 13, and inputs findings to create the report 23 on a request basis. The key image, that is, the reduced image of the examination image 21 corresponding to each finding is attached to the report 23 and stored in the report DB 24.

When the creation of the report 23 is completed, a notice of completion is sent from the report creation terminal 13 to the clinical terminal 11. The doctor accesses the report DB 24 via an address contained in the notice of completion and retrieves the report 23. Thereafter, the report display screen 55 is output to the display 37a of the clinical terminal 11, and the image display screen 56 that displays the examination images 21 related to the report 23 is output to each of the display 37b and 37c.

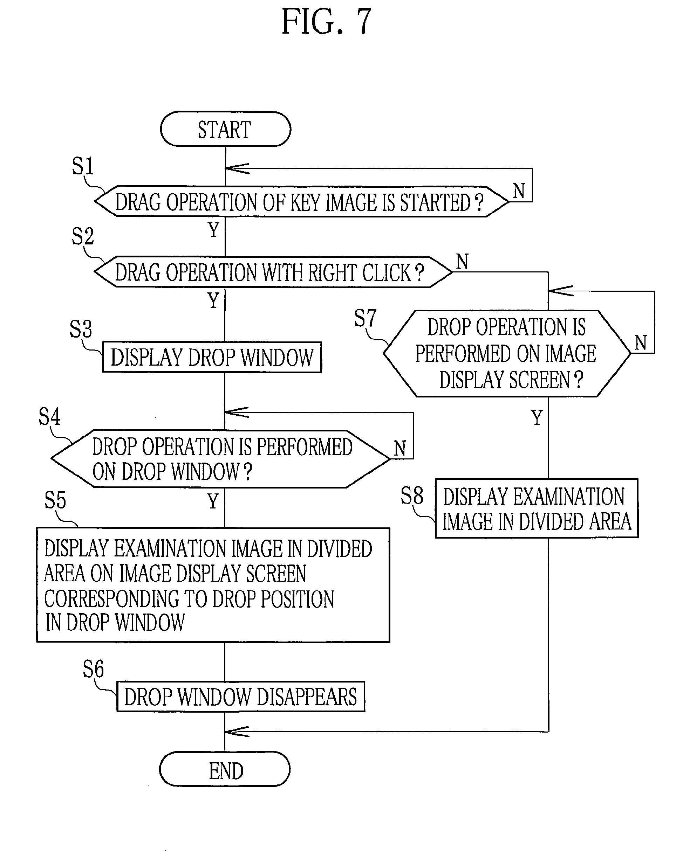

The doctor who has issued the request views the findings and the key image displayed on the report display screen 55. To check the key image in detail, the doctor operates the mouse 42 to drag and drop the key image into the drop window 60, and thus the examination image 21 corresponding to the key image is displayed on the image display screen 56. Hereinafter, with reference to a flowchart in Fig. 7, this drag and drop operation is described.

To drag the key image, the cursor C is placed on the key image with the operation of the mouse 42, and then the mouse 42 is moved with the left click button 42a or the right click button 42b pressed (clicked). When this drag operation of the key image is started (YES in step S1 in Fig. 7), the drag and drop processor 52 judges whether it is a drag operation using the right click button 42b or not (step S2).

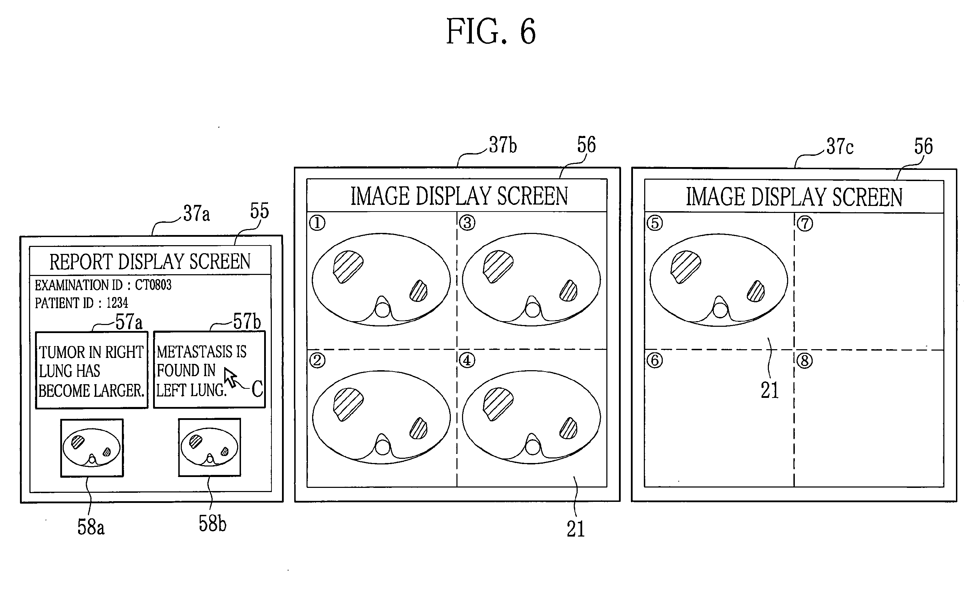

When it is the drag operation using the right click button 42b (YES in the step S2), the drop window display controller 53 displays the drop window 60 in the vicinity of the cursor C on the report display screen 55 as shown in Fig 5 (step S3). The drop window 60 displays reduced divided areas No. 1 to No. 8 of the image display screens 56. When the drop operation is performed, namely, when the right click button 42b is released on the drop window 60 (YES in step S4), the drop position controller 54 detects the divided area of the drop window 60 where the key image has been dropped. In response to this, as shown in Fig. 6, the image display controller 51 displays the examination image 21 corresponding to the dropped key image (the key image 58a in Fig. 6) in the drop position (the divided area No. 5 in Fig. 6) on the image display screen 56 (step S5). The drop window 60 is hidden (step S6) when the examination image 21 is displayed.

When the key image is dragged onto the drop window 60, the pseudo cursor D is displayed on the image display screen 56 to clearly indicate the actual drop position to the operator. The pseudo cursor D is hidden when the drop window 60 is hidden in the step S6.

On the other hand, when it is judged that the drag operation is performed with the click of the left click button 42a ("NO" in the step S2), the drop window 60 is not displayed. In this case, the drop target area or active area is one of the divided areas No.1 to No.8 in the image display screen 56 (step 7). When the drop operation is performed, namely, when the left click button 42a is released on the image display screen 56 (YES in step S7), the examination image 21 corresponding to the key image is displayed in one of the divided areas No. 1 to No. 8 where the key image has been dropped (step S8).

In the indirect drag and drop operation, as described above, the drop window 60 representing the drop target areas is displayed in the vicinity of the cursor C when the drag and drop operation of an object such as the key image is performed. The drop operation is performed on the drop window 60, and thereby the object is actually dropped into the drop target area. The indirect drag and drop operation needs a shorter mouse drag distance than the normal direct drag and drop operation. As a result, eye movements are reduced. Since the indirect drag and drop operation is the same operation as the normal drag and drop operation except that the drop window 60 is displayed, the indirect drag and drop operation has good operability. Thus, the mouse drag distance is reduced without impairing the operability of the drag and drop operation.

In the above embodiment, the displays 37b and 37c on which the image display screens 56 are displayed have higher resolution than the display 37a on which the report display screen 55 is displayed. Accordingly, when the mouse 42 is moved across the report display screen 55 and the image display screen 56 at a constant speed, a moving speed of the cursor C changes, namely, slows down in the image display screen 56. In addition, the cursor C does not move smoothly from the image display screen 56 to the report display screen 55. These make the operation awkward. On the other hand, in the indirect drag and drop operation using the drop window 60, the drop window 60 is displayed on the report display screen 55. There is no need to move the cursor C across the report display screen 55 and the image display screen 56, eliminating the awkwardness. Thus, the present invention is especially effective in medical information systems in which displays with different resolutions are normally used.

In the above embodiment, the drag and drop operation is switched between the direct drag and drop operation and the indirect drag and drop operation using the drop window 60 by the click of the left click button 42a and the click of the right click button 42b. Instead, other switching method may be used. For example, the mode of the drag and drop operation can be switched by an instruction from an operation input device such as the keyboard 41. Alternatively, the indirect drag and drop operation using the drop window 60 can be employed singly.

In the above embodiment, the drop window 60 is displayed as a pop-up window in response to the start of the drag operation. Instead, the drop window 60 may be displayed constantly. A position to display the drop window 60 is not particularly limited. The drop window 60 may be displayed in a fixed position regardless of the position of the cursor C as long as the drop window 60 is located closer to the object (key image) than the image display screen 56 that is the drop target area.

In the above embodiment, each of the image display screens 56 on the displays 37b and 37c is divided into four divided areas as the drop target areas. However, the number of the divided areas may be changed as necessary. Each image display screen 56 may not necessarily be divided into the divided areas, and may be used as a single drop target area. The number' of the displays used for displaying the examination images is not limited to two. One or more than three displays may be used.

In the above embodiment, the drop window 60 is a reduced image or a reduced application window of the image display screens 56. However, the form of the drop window 60 may be changed as necessary.

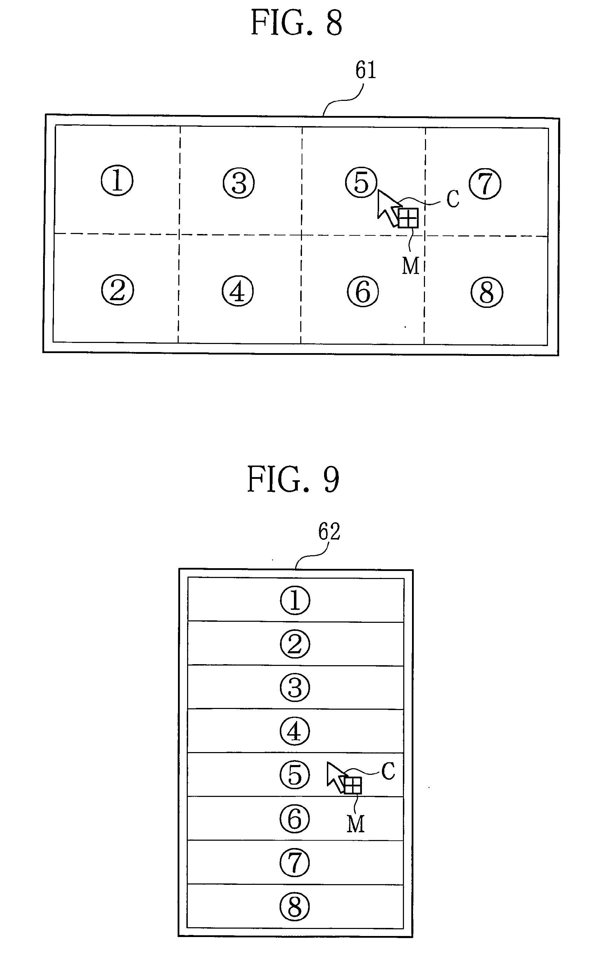

A drop window 61 shown in Fig. 8 is a modified example of the drop window 60. The drop window 61 simply shows the configuration of the divided areas in the image display screens 56, namely, the drop window 61 only displays the numbers assigned to the divided areas in corresponding positions, but not the key images (examination images 21). In Fig. 9, a drop window 62 only shows a list of the numbers assigned to the divided areas without their relative positions. The drop window 62 may be displayed as a context menu. For example, the drop window 62 may be displayed with a click of the right click button 42b at an arbitrary position on the screen. Instead of the numbers, symbols or patterns may be assigned to the divided areas for the sake of identification. For example, the images displayed in the divided areas can be reduced to icons, and such icons can be assigned to the divided areas.

In the above embodiment, the drop window 60 is displayed on the report display screen 55. The drop window 60 may be displayed on the image display screen 56.

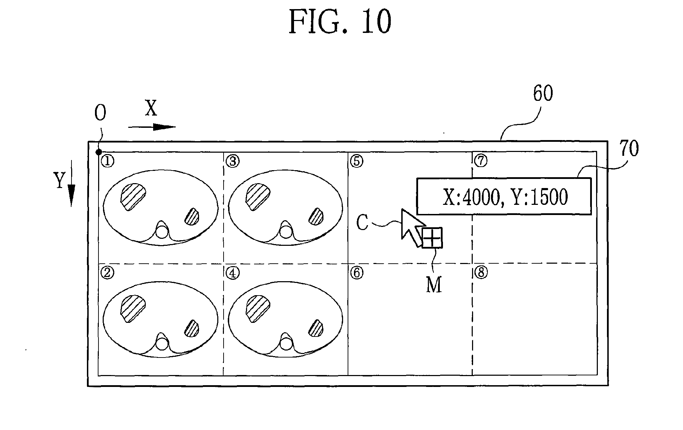

In the above embodiment, when the key image is dragged into the drop window 60, the pseudo cursor D is displayed in the corresponding position on the image display screen 56 to clearly indicate the actual target location for dropping. Alternatively or in addition, the target location may be indicated by coordinates. For example, as shown in Fig. 10, coordinates 70 indicating the actual target location are displayed in the vicinity of the cursor C during the drag operation. The coordinates 70 are the X and Y coordinates from the origin O (an upper left corner of the divided area No. 1) of the combined area of the divided areas of the image display screens 56. The position of the origin O may be changed as necessary. The coordinates of the target location may be represented on a display-by-display basis. In this case, information for display identification is displayed in addition to the coordinates, for example " (X: 1000, Y: 1500) in the second display".

In the above embodiment, the clinical terminal 11 has a multi-display configuration with the three displays 37a to 37c. Alternatively, the present invention can be applied to a case where the report display screen 55 and the image display screen 56 are displayed on the same display, for example, the clinical terminal 11 having a single display.

In the above embodiment, a medical information system is described as an example. The present invention is applicable to any apparatus as long as an object on the computer screen can be drag-and-dropped. The term "object" includes any item on the computer that can be drag-and-dropped, not only files and folders, but also a part of a character string and the like. An object may be drag-and-dropped within the same application window or across different application windows.

In the above embodiment, the drag and drop control method of the present invention is performed using an application program. The drag and drop control method may be performed using an operating system program. In this case, the present invention can be performed only by installing the program on the computer. It should be noted that the present invention is not limited to the program, and may be realized by hardware. Thus, the present invention covers a form of a user interface, a form of a program, a form of an apparatus and recording media for recording the program.

In the above embodiment, the mouse 42 is described as an example of the operation input device for performing the drag and drop operation. Various operation input devices such as a joystick and a keyboard incorporated with a trackball or a touch sensitive pad can be used as long as it has a pointer for moving a cursor and a button for selecting and releasing an object.

Various changes and modifications are possible in the present invention and may be understood to be within the present invention.

## Claims

1. An apparatus for controlling a drag and drop operation comprising:
a display section (37) for displaying an object (58a, 58b);
a drop window display controller (53) for displaying a drop window (60) on said display section, said drop window corresponding to an actual drop target area (56) into which said object is to be dropped, said drop window having a smaller size than said actual drop target area, said drop window being displayed in a position closer to said object than said actual drop target area;
an operating section (38) for instructing holding, dragging and dropping of said object;
a drag and drop processor (52) for dragging said object and dropping said object in said drop window based on an operation of said operating section; and
a drop position controller (54) for controlling a corresponding position in said actual drop target area into which said object is to be dropped based on a drop position of said object in said drop window.

2. The apparatus of claim 1, wherein said object is held, dragged and dropped via a cursor displayed on said display section.

3. The apparatus of claim 2, wherein said object dropped in said actual drop target area is displayed in a large size.

4. The apparatus of claim 2, wherein said drop window display controller displays said drop window when said operating section provides a predetermined instruction.

5. The apparatus of claim 3, wherein said drop window display controller displays said drop window when said object is held, and hides said drop window when said object is dropped in said drop window.

6. The apparatus of claim 3, wherein said display section has multiple displays (37a, 37b, 37c) placed side by side, and said actual drop target area is displayed across said displays, and said drop window is displayed on one of said displays where said object is displayed.

7. The apparatus of claim 6, wherein said actual drop target area has a plurality of divided areas.

8. The apparatus of claim 7, wherein said drop window is a reduced image of said actual drop target area.

9. The apparatus of claim 8, further including a position information display section (51) for displaying position information indicating a position in said actual drop target area, and corresponding to a position in said drop window into which said object is to be dropped.

10. The apparatus of claim 9, wherein said position information display section displays a pseudo cursor in said actual drop target area on said position indicated by said position information.

11. The apparatus of claim 10, wherein said position information is coordinates.

12. A method for controlling a drag and drop operation comprising the steps of:
displaying an object on a display section (37);
displaying a drop window (60) on said display section, said drop window corresponding to an actual drop target area (56) into which said object is to be dropped, said drop window having a smaller size than said actual drop target area, said drop window being displayed in a position closer to said object than said actual drop target area;
operating an operating section (38) to instruct holding, dragging and dropping of said object;
dragging said object and dropping said object inside said drop window based on said operation of said operating section;
and
dropping said object in a corresponding position in said actual drop target area based on a drop position of said object in said drop window.

13. A drag and drop control program executed in a computer, comprising:
a first display function for displaying an object (58a, 58b) on a display section (37);
a second display function for displaying a drop window (60) on said display section, said drop window corresponding to an actual drop target area (56) into which said object is to be dropped, said drop window having a smaller size than said actual drop target area, said drop window being displayed in a position closer to said object than said actual drop target area;
an instruction function for instructing holding, dragging and dropping of said object by operating an operating section (38) ;
a drag and drop function for dragging said object and dropping said object inside said drop window based on said operation of said operating section; and
a drop function for dropping said object in a corresponding position in said actual drop target area based on a drop position of said object in said drop window.

14. A computer terminal (11) comprising:
a computer main unit (40) for executing said program for controlling said drag and drop operation of claim 13;
said operating section; and
said display section (37) on which said object is displayed.

15. The computer terminal of claim 14, wherein said drop window is displayed on said display section when a predetermined instruction is provided by said operating section.
